(19) Europäisches Patentamt
European Patent Office
Office européen des brevets

(11) Veröffentlichungsnummer: **0 122 490**
**A2**

(12) # EUROPÄISCHE PATENTANMELDUNG

(21) Anmeldenummer: **84103001.8**

(22) Anmeldetag: **19.03.84**

(51) Int. Cl.³: **C 07 C 172/00**
C 07 C 35/32, C 07 C 49/633
C 07 C 91/14, C 07 C 121/46
C 07 C 135/00, C 07 D 261/02
C 07 F 7/18

(30) Priorität: **21.03.83 US 477059**

(43) Veröffentlichungstag der Anmeldung:
**24.10.84 Patentblatt 84/43**

(84) Benannte Vertragsstaaten:
**BE CH DE FR GB IT LI NL SE**

(71) Anmelder: **F. HOFFMANN-LA ROCHE & CO. Aktiengesellschaft**

**CH-4002 Basel(CH)**

(72) Erfinder: **Baggiolini, Enrico Giuseppe**
**30 Evergreen Drive**
**North Caldwell, N.J.(US)**

(72) Erfinder: **Boris, Alfred**
**25 Lord Sterling Drive**
**Parsippany, N.J.(US)**

(72) Erfinder: **Batcho, Andrew David**
**19 White Oak Drive**
**North Caldwell, N.J.(US)**

(72) Erfinder: **Uskokovic, Milan Radoje**
**253 Highland Avenue**
**Upper Montclair, N.J.(US)**

(74) Vertreter: **Lederer, Franz, Dr. et al,**
**Patentanwälte Dr. Lederer Franz Meyer-Roxlau Reiner F.**
**Lucile-Grahn-Strasse 22**
**D-8000 München 80(DE)**

(54) **Verfahren zur Herstellung von 1-alpha, 25-Dihydroxyergocalciferol.**

(57) Die Erfindung betrifft ein Verfahren zur Herstellung des 1α,25-Dihydroxyergocalciferols ausgehend von einer Verbindung der Formel

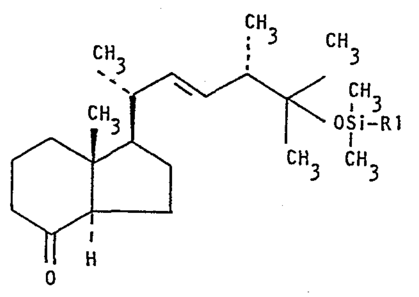

II

worin $R_1$ nieder-Alkyl, Aryl oder Aryl-nieder-alkyl ist, sowie in diesem Verfahren vorkommende neue Zwischenprodukte und praktisch reines kristallines 1α,25- Dihydroxyergocalciferol. Das Endprodukt 1α,25- Dihydroxyergocalciferol kann bei der Behandlung von Zuständen die durch zu tiefe 1α,25-Dihydroxycholecalciferol-Spiegel charakterisiert sind, verwendet werden.

EP 0 122 490 A2

Croydon Printing Company Ltd.

0122490

F.HOFFMANN-LA ROCHE & CO.Aktiengesellschaft, Basel/Schweiz

RAN 4212/39

# Verfahren zur Herstellung von 1α,25-Dihydroxyergocalciferol

Die Erfindung betrifft ein Verfahren zur Herstellung des 1α,25-Dihydroxyergocalciferols der Formel

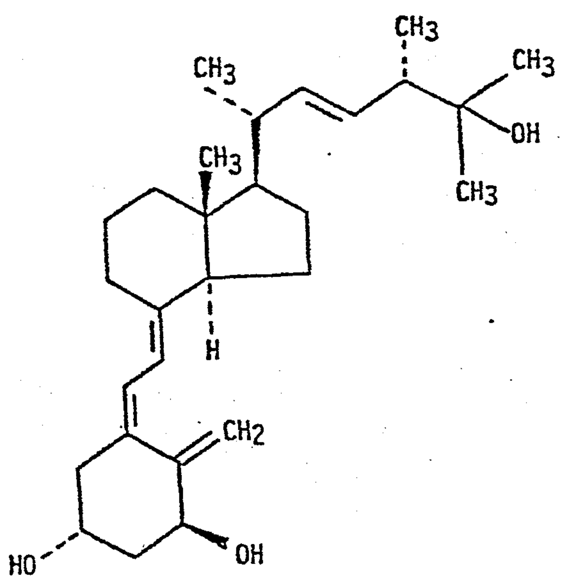

I

welches dadurch gekennzeichnet ist, dass man

a) eine Verbindung der Formel

Mé/ 25.1.84

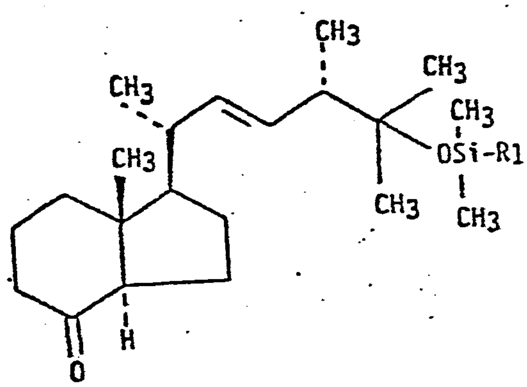

II

worin $R_1$ nieder-Alkyl, Aryl oder Aryl-nieder-alkyl ist, mit einer Verbindung der Formel

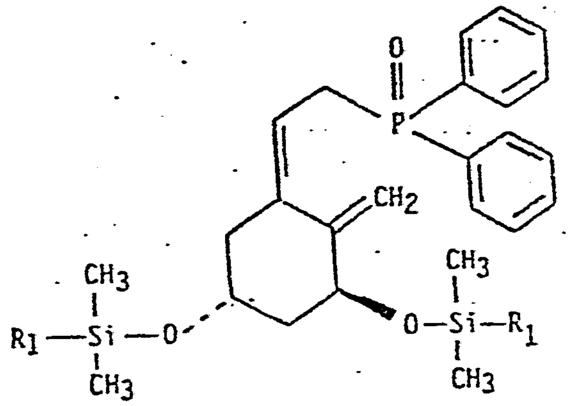

III

worin $R_1$ die obige Bedeutung hat, zu einer Verbindung der Formel

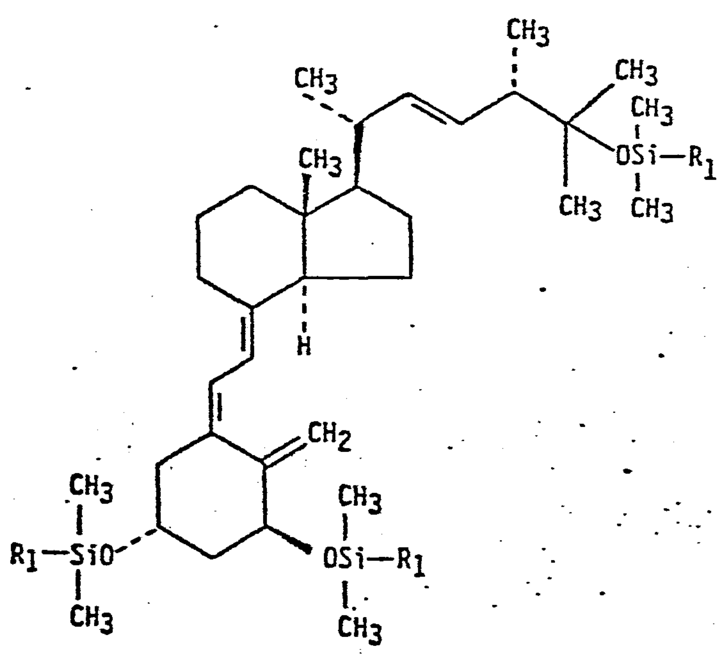

IV

worin $R_1$ die obige Bedeutung hat, umsetzt und

b) die Schutzgruppen aus der Verbindung der Formel IV abspaltet.

Im Rahmen der Erfindung bedeutet der Ausdruck "nieder-Alkyl" eine geradkettige oder verzweigte Kohlenwasserstoffgruppe mit 1-8 C-Atome, z.B. Methyl, Aethyl, n-Propyl, i-Propyl, t-Butyl, Hexyl, Heptyl und Octyl. Der Ausdruck "Aryl-nieder-alkyl" betrifft eine durch eine Arylgruppe substituierte nieder-Alkylgruppe, z.B. Benzyl, Phenäthyl und Phenylpropyl. Der Ausdruck "Aryl" bezeichnet eine Gruppe, die von einem aromatischen Kohlenwasserstoff, der durch einen oder mehreren Alkylresten substituiert sein kann, abgeleitet ist, z.B. Phenyl und p-Tolyl.

Ein Keton der Formel II kann mit einem Phosphinoxyd der Formel III in Gegenwart einer Base in einem herkömmlichen Aetherlösungsmittel, z.B. Diäthyläther oder Tetrahydrofuran, unter einer inerten Atmosphäre bei einer Temperatur im Bereich von etwa -80 bis -50°C, umgesetzt werden. Beispiele von Basen sind nieder-Alkyllithiumverbindungen oder Alkalimetalldialkyl- oder -disilylamide. Die Verbindung der Formel IV kann durch Elutionschromatographie auf Silicagel gereinigt werden.

Die Verbindung der Formel IV kann in eine Verbindung der Formel I durch Reaktion mit einem Tetraalkylammoniumfluorid, z.B. Tetrabutylammoniumfluorid, in einem inerten organischen Lösungsmittel, wie einem nieder-aliphatischen Kohlenwasserstoff, z.B. Hexan oder Heptan, einem aromatischen Kohlenwasserstoff, z.B. Benzol oder Toluol, oder einem Aether, z.B. Diäthyläther oder Tetrahydrofuran, vorzugsweise in Tetrahydrofuran, übergeführt werden. Diese Reaktion kann bei einer Temperatur im Bereich von etwa 0°C bis Siedepunkt des Lösungsmittels, vorzugsweise bei Raumtemperatur, durchgeführt werden.

Eine Verbindung der Formel II kann wie folgt hergestellt werden:

Eine Sulfonyloxyverbindung der Formel

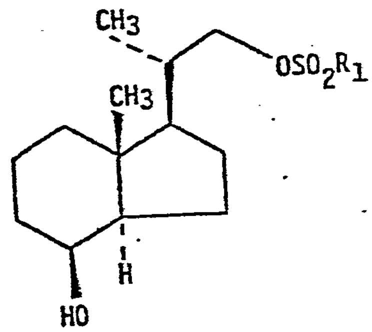

V

worin $R_1$ nieder-Alkyl, Aryl oder Aryl-nieder-alkyl ist, wird mit Natriumcyanid zu einer Verbindung der Formel

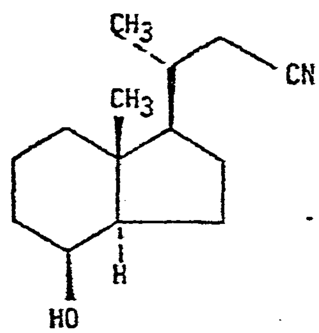

VI-A

umgesetzt.

Diese Reaktion wird vorzugsweise in einem inerten organischen Lösungsmittel, wie Aceton, Acetonitril, Dimethylformamid oder Dimethylsulfoxid, bei einer Temperatur im Bereich von etwa 0°C bis Siedepunkt des Lösungsmittel durchgeführt.

Die Verbindung der Formel VI-A wird durch Behandlung mit einem Hydrid, z.B. Lithiumaluminiumhydrid oder vorzugsweise Diisobutylaluminiumhydrid, in einem inerten organischen Lösungsmittel, wie einem nieder-aliphatischen Kohlenwasserstoff, einem Aether oder einem aromatischen Kohlenwasserstoff, z.B. einem der oben angegebenen Lösungsmittel oder einem nieder-Alkylhalogenid, z.B. Methylenchlorid, Chloroform oder Tetrachlorkohlenstoff, zu einem Aldehyd der Formel

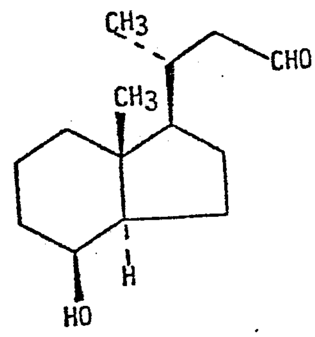

VI-B

reduziert.

Die Reaktion wird bei einer Temperatur im Bereich von etwa -70 bis 80°C, vorzugsweise von 0°C bis Raumtemperatur, durchgeführt.

Die Verbindung der Formel VI-B wird mit einem Alkyl-, Aryl- oder Aryl-nieder-alkyl-hydroxylamin-hydrochlorid in Gegenwart eines tertiären Amins, wie eines Tri-nieder-alkylamins, z.B. Pyridin, bei einer Temperatur im Bereich von etwa 0-80°C, vorzugsweise bei Raumtemperatur, zu einer Verbindung der Formel

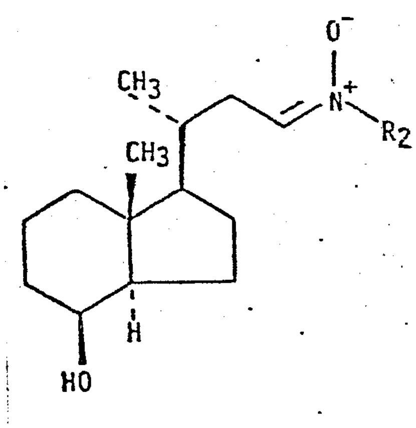

VII

worin $R_2$ nieder-Alkyl, Aryl oder Aryl-nieder-alkyl ist, umgesetzt.

0122490

Die Reaktion wird zweckmässigerweise in einem Lösungsmittel, wie einem nieder-Alkanol, z.B. Methanol, Aethanol, t-Butanol oder Isopropanol, oder einem Aether, z.B. Diäthyläther oder Tetrahydrofuran, durchgeführt.

Eine Verbindung der Formel VII wird durch Reaktion mit einem Alkyl-, Aryl- oder Aryl-nieder-alkyl-3,3-dimethylacrylat in einem inerten organischen Lösungsmittel bei einer Temperatur im Bereich von etwa Raumtemperatur bis Siedepunkt des Lösungsmittels, vorzugsweise bei etwa 140°C, zu einer Verbindung der Formel

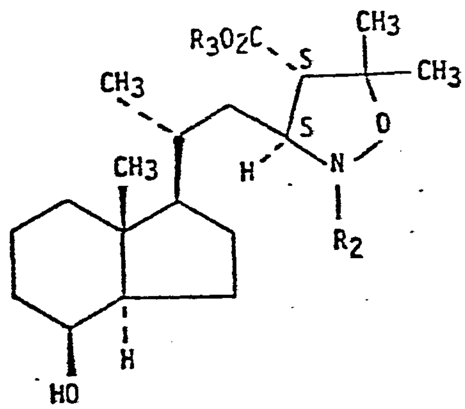

VIII

worin $R_3$ nieder-Alkyl, Aryl oder Aryl-nieder-alkyl ist und $R_2$ die obige Bedeutung hat,

cyclisiert.

Beispiele von 3,3-Dimethylacrylaten sind Methyl-, Aethyl- oder Benzyl-3,3-dimethylacrylat. Beispiele von Lösungsmitteln sind nieder-aliphatische Kohlenwasserstoffe, nieder-Alkanole, Aether und aromatische Kohlenwasserstoffe, z.B. die weiter oben zitierten Lösungsmittel.

Eine Verbindung der Formel VIII wird durch Reaktion mit einem Hydrid in einem inerten organischen Lösungsmittel zu einer Verbindung der Formel

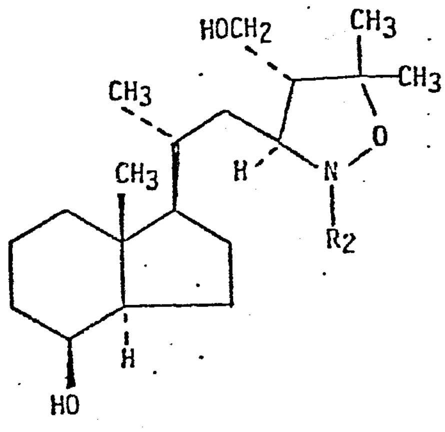

IX

worin $R_2$ die obige Bedeutung hat, reduziert.

Beispiele von Hydriden und inerten organischen Lösungsmitteln sind weiter oben angegeben. Die Reduktion wird bei einer Temperatur im Bereich von etwa -70 bis 80°C, vorzugsweise bei Raumtemperatur, durchgeführt.

Eine Verbindung der Formel IX wird mit einem Sulfonierungsmittel, z.B. Methansulfonylchlorid oder vorzugsweise p-Toluolsulfonylchlorid, in einem basischen organischen Lösungsmittel, z.B. einem niederen tertiären Alkylamin, Pyridin oder substituiertes Pyridin, zu einer Verbindung der Formel

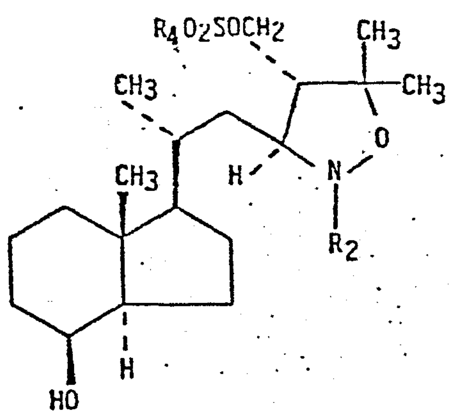

X

worin $R_4$ nieder-Alkyl, Aryl oder Aryl-nieder-alkyl ist und $R_2$ die obige Bedeutung hat, umgesetzt.

Die Reaktion wird bei einer Temperatur im Bereich von etwa 0-80°C, vorzugsweise bei Raumtemperatur, durchgeführt.

Eine Verbindung der Formel X wird durch Reaktion mit einem Hydrid in einem inerten organischen Lösungsmittel zu einer Verbindung der Formel

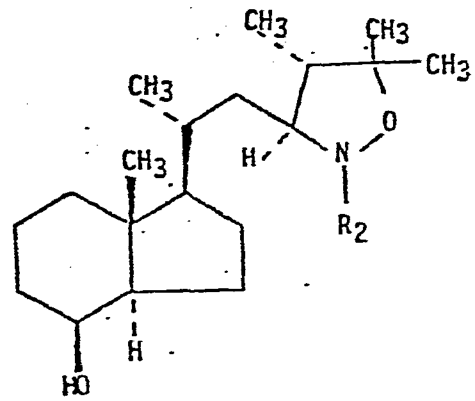

XI

worin $R_2$ die obige Bedeutung hat, reduziert.

Beispiele von Hydriden und inerten organischen Lösungsmitteln sowie die Reaktionstemperatur sind weiter oben angegeben.

Eine Verbindung der Formel XI wird in eine Verbindung der Formel

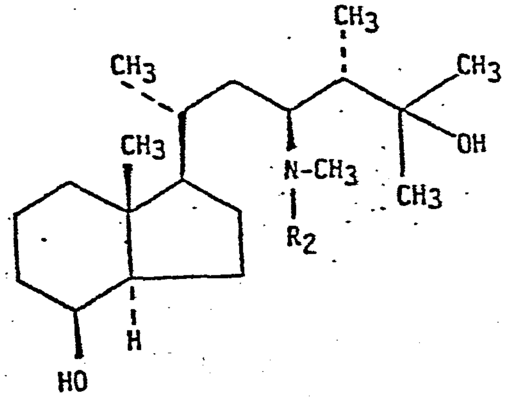

XII

worin $R_2$ die obige Bedeutung hat, durch Behandlung mit Methyljodid in trockenem Toluol bei

einer Temperatur im Bereich von etwa Raumtemperatur bis 80°C, vorzugsweise bei 60°C, umgewandelt. Diese Reaktion wird durch Reduktion mit Zinkstaub in wässriger Essigsäure bei Raumtemperatur gefolgt.

Eine Verbindung der Formel XII wird in eine Verbindung der Formel

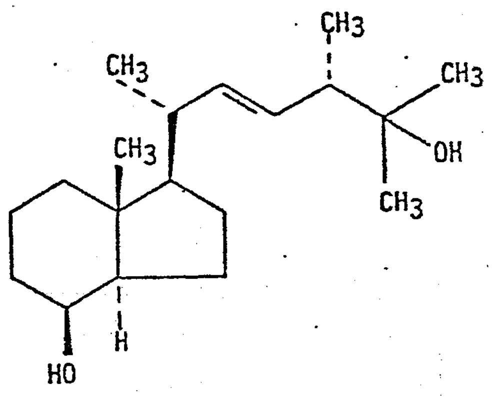

XIII

durch eine Eliminationsreaktion umgewandelt. Diese Reaktion besteht darin, dass man eine Verbindung der Formel XII zuerst mit Methyljodid in Toluol bei einer Temperatur im Bereich von etwa Raumtemperatur bis 100°C, vorzugsweise bei 70°C, und dann mit Kalium-t-butoxyd in t-Butylalkohol bei Rückflusstemperatur behandelt.

Eine Verbindung der Formel XIII wird zu einem Keton der Formel

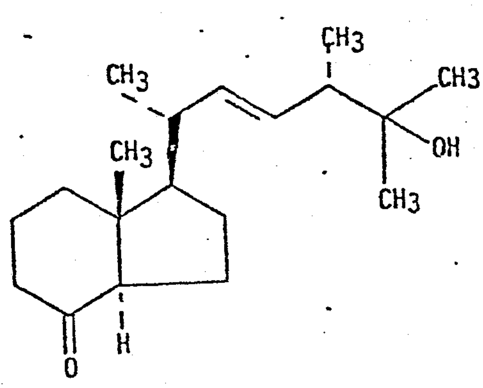

XIV

durch Behandlung mit Pyridiniumchlorochromat in einem inerten organischen Lösungsmittel, wie einem nieder-alipha-

0122490

tischen Kohlenwasserstoff, einem aromatischen Kohlenwasserstoff oder einem nieder-Alkylhalogenid, z.B. einem der weiter oben angegebenen Lösungsmittel, oxidiert. Die Reaktion wird bei einer Temperatur im Bereich von etwa 0°C bis Siedepunkt des Lösungsmittels, vorzugsweise bei Raumtemperatur, durchgeführt.

Die Hydroxylgruppe einer Verbindung der Formel XIV wird durch Reaktion mit einem Silylierungsmittel in einem inerten organischen Lösungsmittel geschützt, wobei man eine Verbindung der Formel

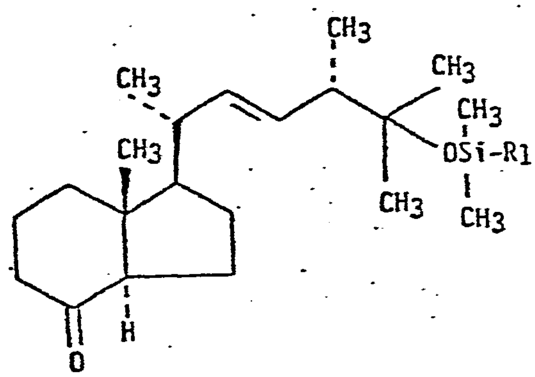

II

worin $R_1$ die obige Bedeutung hat, erhält.

Beispiele von Silylierungsmittel sind Trimethylsilylchlorid, t-Butyldimethylsilylchlorid und vorzugsweise N-(Trimethylsilyl)imidazol. Beispiele von inerten organischen Lösungsmittel sind nieder-aliphatische Kohlenwasserstoffe, Aether, aromatische Kohlenwasserstoffe und nieder-Alkylhalogenide, z.B. eines der weiter oben angegebenen Lösungsmittel. Die Reaktion wird bei einer Temperatur im Bereich von etwa -70 bis 80°C, vorzugsweise bei Raumtemperatur, durchgeführt.

Die Verbindungen der Formeln II, VI-A, VI-B und VII bis XII sowie das praktisch reine kristalline 1α,25-Dihydroxyergocalciferol der Formel I sind neu und als solche ebenfalls Gegenstand der vorliegenden Erfindung.

Das 1α,25-Dihydroxyergocalciferol der Formel I kann bei der Behandlung von Zuständen, die durch zu tiefe 1α,25-Dihydroxycholecalciferol-Spiegel in Warmblütern, gekennzeichnet sind, insbesondere bei der Behandlung der Osteoporose und der Osteodystrophie, verwendet werden. Diese Aktivität konnte wie folgt festgestellt werden:

Die antirachitische Aktivität wurde nach dem in A. Boris et al., J. of Nutrition, 197, 1977, 194 beschriebenen Verfahren ermittelt. 1α,25-Dihydroxyergocalciferol wurde in Propylenglykol 21 Tage unter UV-freier Beleuchtung an mit Vitamin D-armem Futter gefütterten Küken oral verabreicht. Es werden 9-10 Küken für jede Behandlungsgruppe verwendet. Es wurden folgende Resultate ermittelt:

Tabelle I

| Dosis ng/Küken/Tag | mittleres Tibiaaschengewicht (mg) |
|---|---|
| 0 | 88,0 ± 6,1 |
| 30 | 103,4 ± 3,5 |
| 100 | 130,0 ± 6,3 |
| 300 | 141,3 ± 5,1 |

Die Verhütung der durch Aethan-1-hydroxy-1,1-diphosphat (EHDP) induzierten Blockierung der Mineralisierung wurde nach dem in A. Boris et al., J. of Nutrition, 108, 1978, 1899 beschriebenen Verfahren ermittelt. 1α,25-Dihydroxyergocalciferol wurde an durch subkutane Injektion von EHDP behandelten Ratten oral verabreicht. Es werden 9-10 Ratten für jede Behandlungsgruppe verwendet. Die folgenden Resultate wurden ermittelt:

Tabelle II

| Dosis ng/Ratte/Tag | mittlere Breite der Tibiaepiphysenplatte (μ) |
|---|---|
| 0 | 1497 ± 20 |
| 1 | 1471 ± 34 |
| 3 | 1409 ± 29 |
| 10 | 1233 ± 29 |
| 30 | 933 ± 15 |
| Vehikel-Kontrollen | 441 ± 5 |

Die Verbindung der Formel I kann in Tagesdosen im Bereich von etwa 1-5, vorzugsweise 2 mg, zur Behandlung der Osteoporose und der Osteodystrophie verabreicht werden. Die Verbindung der Formel I kann auch subkutan, intramuskulär, intravenös oder intraperitoneal verabreicht werden.

Die Verbindung der Formel I kann in der Herstellung von Tabletten, Kapseln oder Elixiren zur oralen Verabreichung oder von sterilen Lösungen oder Suspensionen für die parenterale Verabreichung verwendet werden. Etwa 1-5 mg der Verbindung der Formel I werden mit einem pharmazeutisch anwendbaren Vehikel, Träger, Excipient, Bindemittel, Konservierungsmittel, Stabilisierungsmittel oder Geschmackstoff in herkömmlichen pharmazeutischen Einzeldosen vermischt.

Beispiele von Hilfsmittel, die in Kapseln und dergleichen verwendet werden können, sind ein Bindemittel, wie Tragantgummi, Akazie, Maisstärke oder Gelatine, ein Excipient, wie Dicalciumphosphat, ein Versetzungsmittel, wie Maisstärke, Kartoffelstärke oder Alginsäure, ein Gleitmittel, wie Magnesiumstearat, ein Süsstoff, wie Sucrose, Lactose oder Saccharin, ein Geschmacksmittel, wie Pfefferminz, Wintergrünöl oder Kirsche. Es können andere Materialien, wie Ueberzüge zur Modifizierung des Aspektes der Einzeldosen verwendet werden. Tabletten können z.B. mit

Shellack, Zucker oder beide überzogen werden. Ein Sirup oder Elixir kann die Aktivsubstanz, Sucrose als Süssmittel, Methyl- und Propylparaben als Konservierungsmittel, einen Farbstoff und einen Geschmackstoff, wie Orangen- oder Kirschen-Aroma enthalten.

Sterile Präparate zum Injizieren können in üblicher Weise dadurch hergestellt werden, dass man die Aktivsubstanz in einem Vehikel, wie Injektionswasser, einem in der Natur vorkommenden pflanzlichen Oel, wie Sesamöl, Kokosnussöl, Erdnussöl oder Baumwollsamenöl, oder einem synthetischen fetten Bindemittel, wie Aethyloleat, auflöst oder suspendiert. Es können auch Puffer, Konservierungsmittel und Antioxidantien zugesetzt werden.

Die folgenden Beispiele veranschaulichen die Erfindung. Alle Temperaturen sind in °C.

Beispiel 1

Einer Lösung von 6,75 g (137,5 mMol) Natriumcyanid in 175 ml trockenem Dimethylsulfoxid (DMSO) wird unter einer Argonatmosphäre eine Lösung von 42,0 g (114,6 mMol) [1R-[1α(S*),3aβ,4α,7aα]]-Octahydro-4-hydroxy-β,7a-dimethyl-1H-inden-1-äthanol-α-(4-methylbenzolsulfonat) in 100 ml trockenem DMSO zugesetzt. Die entstandene Lösung wird 2 1/4 Stunden bei 90° erhitzt, dann in 2,5 l Kochsalzlösung gegossen und mit 6 x 500 ml Aether extrahiert. Jeder Extrakt wurde mit 500 ml Salzlösung gewaschen. Die vereinigten organischen Phasen wurden getrocknet und eingedampft, wobei man 25,60 g rohes Produkt erhielt. Nach Reinigung durch Chromatographie auf Silicagel mit Hexan/Aethylacetat erhielt man 22,26 g (80% Ausbeute) [1R-[1α(R*),-3aβ,4α,7aα]]-Octahydro-4-hydroxy-β,7a-dimethyl-1H-inden-1-propannitril. Eine analytische Probe wurde aus Aether/Petroläther kristallisiert, Schmelzpunkt 90,5-91,5°; $[\alpha]_D^{25}$ +46,66 (c 0,6986, Chloroform).

Beispiel 2

Einer Lösung von 11,55 g (52,28 mMol) des Produktes von Beispiel 1 in 300 ml trockenem Toluol wurden bei 0° 73 ml (109,6 mMol) 1,5M Diisobutylaluminiumhydrid in Toluol zugesetzt. Das Gemisch wurde 1 Stunde gerührt. Die Reaktion wurde durch Zusatz von 2 l kaltem 2N Salzsäure beendet. Das Rühren wurde fortgesetzt und 560 ml Aether wurden zugesetzt. Die Phasen wurden getrennt und die wässrige Phase wurde mit 3 x 500 ml Aether extrahiert. Die vereinigten organischen Schichten wurden getrocknet und man erhielt 12,99 g rohes [1R,[1α(S*),3aβ,4α,7aα]]-Octahydro-4-hydroxy-β,7a-dimethyl-1H-inden-1-propanal.

Beispiel 3

Einer Lösung von 3,81 g des rohen Produktes von Beispiel 2 in 100 ml trockenem Methylenchlorid wurden 1,56 g

(18,7 mMol) N-Methylhydroxylamin-hydrochlorid zugesetzt. Die Suspension wurde bei Raumtemperatur gerührt, dann wurden 3,20 ml (23,2 mMol) Triäthylamin zugesetzt. Die Lösung wurde 19 Stunden bei Raumtemperatur gerührt, dann mit 2 x 50 ml 5%igem Natriumbicarbonat extrahiert. Die organische Phase wurde getrocknet und eingedampft und man erhielt 3,78 g (88% Ausbeute) [1R-[1α(R*,Z),3aβ,4α,7aα]]-Octahydro-7a-methyl-1-[1-methyl-3-(methylimino)propyl]-1H-inden-4-ol-N-oxid. Eine analytische Probe wurde aus Aethylacetat kristallisiert, Schmelzpunkt 145-146°; $[\alpha]_D^{25}$ +41,45° (c 0,808, Chloroform).

## Beispiel 4

Eine Lösung von 7,50 g (29,6 mMol) des Produkts von Beispiel 3 und 10,13 g (89 mMol) Methyl-3,3-dimethylacrylat in 75 ml Xylol wurde unter Stickstoffatmosphäre im Oelbad bei 140° während 15 Stunden erhitzt. Nach Eindampfen unter vermindertem Druck erhielt man 11,6 g rohes Produkt. Chromatographie auf Silicagel mit Methylenchlorid-Aethylacetat ergab 5,36 g unreines Produkt. Wiederholte Chromatographie ergab 4,5 g (41% Ausbeute) reines [3S-[3β,4α,3-[(2R*),1R*(1β,3aα,4β,7aβ)]]]-3-[2-(Octahydro-4-hydroxy-7a-methyl-1H-inden-1-yl)propyl]-2,5,5-trimethyl-4-isoxazolidincarbonsäure-methylester. Eine analytische Probe hatte einen Schmelzpunkt von 80-81° (aus Hexan), $[\alpha]_D^{25}$ +102,6 (c 1,05, Chloroform).

## Beispiel 5

Einer Lösung von 3,0 g (8,2 mMol) des Produktes von Beispiel 4 in 100 ml trockenem Tetrahydrofuran wurden unter Argonatmosphäre 930 mg (24,5 mMol) Lithiumaluminiumhydrid zugesetzt. Nach Rühren während 3,5 Stunden bei Raumtemperatur wurde die Suspension im Eisbad abgekühlt und 1,0 ml Wasser gefolgt von 1,5 ml 1N Natriumhydroxid wurden zugetropft. Nach 15 Minuten wurde die Suspension filtriert und der Filterkuchen wurde mit 2 x 100 ml Aether gewaschen.

Die Filtrate wurden unter vermindertem Druck eingedampft und man erhielt 2,85 g Rückstand. Chromatographie auf Silicagel mit Aethylacetat ergab 2,49 g (90% Ausbeute) [3S-[3β,4α,3-[(2R*),1R*(1β,3aα,4β,7aβ)]]]-3-[2-(Octahydro-4-hydroxy-7a-methyl-1H-inden-1-yl)propyl]-2,5,5-trimethyl-4-isoxazolidinmethanol. Eine analytische Probe hatte einen Schmelzpunkt von 153° (Acetonitril), $[\alpha]_D^{25}$ +106,4° (c 1,07, Chloroform).

## Beispiel 6

Einer Lösung von 2,49 g (7,33 mMol) des Produkts von Beispiel 5 in 75 ml trockenem Pyridin wurden unter Rühren unter Argonatmosphäre im Eisbad bei 5° 2,10 g (11,0 mMol) p-Toluolsulfonylchlorid während 10 Minuten zugesetzt. Das Eisbad wurde entfernt und das Reaktionsgemisch wurde 48 Stunden bei Raumtemperatur gerührt, dann auf 5° abgekühlt und 5,0 ml Wasser wurden zugesetzt. Nach 30 Minuten wurde das Reaktionsgemisch in 2 l 2N Schwefelsäure geschüttet und dann mit 3 x 150 ml Methylenchlorid extrahiert. Dann wurde mit 50 ml 10% Natriumbicarbonat gewaschen. Die Methylenchloridphasen wurden getrocknet, filtriert und eingedampft und man erhielt 3,28 g (91%) [1R-[1α(R*),3aβ,4α,7aα,(3S*,-4S*)]]-Octahydro-7a-methyl-1-[1-methyl-2-[4-[[[(4-methylphenyl)sulfonyl]oxy]methyl]-2,5,5-trimethyl-3-isoxazolidinyl]äthyl]-1H-inden-4-ol. Eine analytische Probe des Hydrats hatte einen Schmelzpunkt von 114-115° (aus Hexan), $[\alpha]_D^{25}$ +53,3 (c 0,994, Chloroform).

## Beispiel 7

Einer Suspension von 3,28 g (6,65 mMol) des Produkts von Beispiel 6 in 100 ml trockenem Tetrahydrofuran wurden unter Rühren unter Argonatmosphäre 756 mg (19,9 mMol) Lithiumaluminiumhydrid zugesetzt. Die Suspension wurde 1 Stunde bei Rückflusstemperatur erhitzt, über Nacht bei Raumtemperatur belassen, dann im Eisbad abgekühlt. Nach Zusatz von 1,0 ml Wasser und 1,5 ml 1N Natriumhydroxid

wurde die Suspension 30 Minuten gerührt, dann filtriert. Der Filterkuchen wurde mit 2 x 100 ml Aether gewaschen und die vereinigten Filtrate wurden zu 2,48 g Rückstand eingedampft. Chromatographie auf Silicagel mit Hexan-Aethylacetat ergab 1,90 g [1R-[1α(R*),3aβ,4α,7aα,(3S*,4S*)]]-Octahydro-7a-methyl-1-[1-methyl-2-(2,4,5,5-tetramethyl-3-isoxazolidinyl)äthyl]-1H-inden-4-ol in Form eines farblosen Oels.

## Beispiel 8

Eine Lösung von 1,556 g (4,8 mMol) des Produkts von Beispiel 7 und 0,5 ml (1,0 g, 7,2 mMol) Methyljodid in 75 ml trockenem Toluol wurde im Oelbad bei 60° unter Argonatmosphäre 17 Stunden erhitzt. Die Suspension wurde unter vermindertem Druck eingedampft und der Rückstand wurde bei Raumtemperatur mit 100 ml wässriger Essigsäure und 1,72 g (26,3 mMol) Zinkstaub während 2,5 Stunden behandelt. Nach Eindampfen unter vermindertem Druck wurde der Rückstand zwischen 100 ml Aethylacetat und 25 ml 1,5N Ammoniumhydroxid gefolgt von 25 ml Kochsalzlösung aufgetrennt. Die wässrigen Phasen wurden mit 3 x 50 ml Aethylacetat extrahiert. Die Aethylacetatphasen wurden getrocknet, filtriert und eingedampft und man erhielt 1,554 g rohes Produkt. Chromatographie auf Silicagel mit Aethylacetat-Triäthylamin ergab 1,46 g (89% Ausbeute) reines [1R-[1α(R*,S*,S*),3aβ,4α,7aα]]-Octahydro-γ-(dimethylamino)-4-hydroxy-α,α,β,ε,7a-pentamethyl-1H-inden-1-pentanol. Eine analytische Probe hatte einen Schmelzpunkt von 180-181° (aus Aethylacetat), $[\alpha]_D^{25}$ +4,79° (c 1,044, Chloroform).

## Beispiel 9

Eine Lösung von 679 mg (2,0 mMol) des Produkts von Beispiel 8 und 0,35 ml (859 mg, 6,0 mMol) Methyljodid in 10 ml Toluol wurde bei 70° unter einer Argonatmosphäre während 20 Stunden erhitzt. Nach Eindampfen unter vermindertem Druck wurde der Rückstand in 25 ml destilliertem

t-Butylalkohol unter einer Argonatmosphäre gelöst und es wurde Kalium-t-butoxid zugesetzt. Die Suspension wurde 5 Stunden bei Rückflusstemperatur erhitzt, dann auf Raumtemperatur abgekühlt und nach Zusatz von 0,5 ml Wasser zur Trockene eingedampft. Der Rückstand wurde mit 2 x 25 ml Methylenchlorid zu 0,71 g rohem Produkt verrieben. Chromatographie auf Silicagel mit Hexan-Aethylacetat ergab 0,460 mg (78% Ausbeute) [1R-[(1α(R*),2E,4S*,3aβ,4α,7aα]]-Octahydro-1-(5-hydroxy-1,4,5-trimethyl-2-hexenyl)-7a-methyl-1H-inden-4-ol. Eine analytische Probe hatte einen Schmelzpunkt von 103-104° (aus Hexan), $[\alpha]_D^{25}$ +2,01° (c 1,046, Chloroform).

## Beispiel 10

Eine Lösung von 200,0 mg (0,684 mMol) des Produkts von Beispiel 9 in 3 ml Methylenchlorid wurde einer Suspension von 440,0 mg (2,041 mMol) Pyridiniumchlorochromat in 12 ml Methylenchlorid zugesetzt und das entstandene Gemisch wurde während 3 Stunden bei Raumtemperatur gerührt. Es wurde dann mit 40 ml Diäthyläther verdünnt, filtriert und das Lösungsmittel wurde abgedampft. Der Rückstand wurde durch Filtrierung durch Silicagel bereinigt, mit Hexan-Aethylacetat (3:1) eluiert und man erhielt 185,0 mg (93% Ausbeute) [1R-[1α(1R*,2E,4S*),3aβ,7aα]]-Octahydro-1-(5-hydroxy-1,4,5-trimethyl-2-hexenyl)-7a-methyl-4H-inden-4-on, welches aus Hexan-Methylenchlorid kristallisiert wurde, Schmelzpunkt 101-102°, $[\alpha]_D^{25}$ -6,89° (c 0,5, Aethanol).

## Beispiel 11

Eine Lösung von 200,0 mg (0,679 mMol) des Produkts von Beispiel 10 in 10 ml Methylenchlorid wurde mit 0,4 ml (2,726 mMol) N-(Trimethylsilyl)-imidazol behandelt und das entstandene Gemisch wurde während 18 Stunden unter Argon bei Raumtemperatur gerührt. Es wurde dann mit 1 ml Wasser behandelt, während 30 Minuten gerührt, dann mit Methylenchlorid verdünnt. Die organische Phase wurde abgetrennt,

mit Wasser und Kochsalzlösung gewaschen, getrocknet und zur Trockene eingedampft. Der Rückstand wurde durch Filtrieren durch Silicagel bereinigt und mit Hexan-Aethylacetat eluiert und man erhielt 214,0 mg (84% Ausbeute) reines [1R-[1α-(1R*,2E,4S*),3aβ,7aα]]-Octahydro-1-(1,4,5-trimethyl-5-trimethylsilyloxy-2-hexenyl)-7a-methyl-4H-inden-4-on in Form eines dicken farblosen Oels.

## Beispiel 12

Eine Lösung von 360,0 mg (0,617 mMol) [3S-(3α,-5β,Z)]-2-[2-Methylen-3,5-bis-[(1,1-dimethyläthyl)dimethylsilyloxy]cyclohexyliden]äthyldiphenylphosphinoxid in 12 ml Tetrahydrofuran wurde auf -78° abgekühlt und unter Argon mit 0,390 ml (0,604 mMol) einer 1,55M Lösung von n-Butyllithium in Hexan behandelt. Nach Rühren während 5 Minuten wurde eine Lösung von 150,0 mg (0,411 mMol) des Produkts von Beispiel 11 in 3 ml Tetrahydrofuran zugesetzt und das entstandene Gemisch wurde 1 1/2 Stunden bei -78° gerührt. Es wurde dann mit 5 ml eines 1:1-Gemisches von 1N Natriumbicarbonat und 2N Kaliumnatriumtartrat behandelt, auf Raumtemperatur abkühlen gelassen, mit Wasser verdünnt und mit 3 x 80 ml Aethylacetat extrahiert. Die organischen Extrakte wurden mit Kochsalzlösung gewaschen, getrocknet und eingedampft. Der Rückstand wurde durch Chromatographie auf Silicagel mit Hexan-Aethylacetat gereinigt und man erhielt 261,0 mg eines farblosen dicken Oels. Dieses wurde in 6 ml Tetrahydrofuran gelöst, mit 1,8 ml (1,8 mMol) einer 1M-Lösung von Tetrabutylammoniumfluorid in Tetrahydrofuran kombiniert und während 23 Stunden unter Argon bei Raumtemperatur gerührt. Das Lösungsmittel wurde im Vakuum entfernt und der Rückstand wurde in 100 ml Aethylacetat gelöst, mit 5 x 30 ml Wasser und 50 ml Kochsalzlösung gewaschen, getrocknet und eingedampft. Der Rückstand wurde durch Filtrieren durch Silicagel und Elution mit Aethylacetat gereinigt und man erhielt 140,0 mg (80%) reines 9,10-seco(5Z,7E,22E)-5,7,10(19),22-Ergostatetraen-1α,3β,25-triol (1α,25-Dihydroxyergocalciferol); Schmelz-

punkt 165-167°, $[\alpha]_D^{25}$ +47,2° (c 0,2, Aethanol).

Beispiel 13

| Bestandteile | | mg/Kapsel | | |
|---|---|---|---|---|
| 1. | 1α,25-Dihydroxyergocalciferol | 0,001 | 0,0025 | 0,005 |
| 2. | Polyäthylenglykol 400 | 200 | 200 | 200 |
| 3. | butyliertes Hydroxyanisol | 0,1 | 0,1 | 0,1 |
| 4. | Ascorbylpalmitat | 1,0 | 1,0 | 1,0 |

Verfahren:

Die Bestandteile 1, 3 und 4 werden unter einer Stickstoffatmosphäre in dem Bestandteil 2 gelöst und verkapselt.

## Patentansprüche

1. Verfahren zur Herstellung des 1α,25-Dihydroxyergocalciferols, dadurch gekennzeichnet, dass man

a) eine Verbindung der Formel

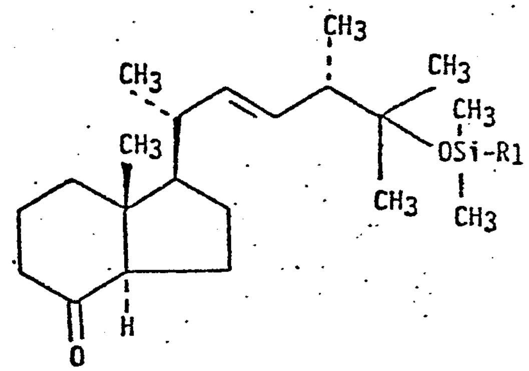

II

worin $R_1$ nieder-Alkyl, Aryl oder Aryl-nieder-alkyl ist, mit einer Verbindung der Formel

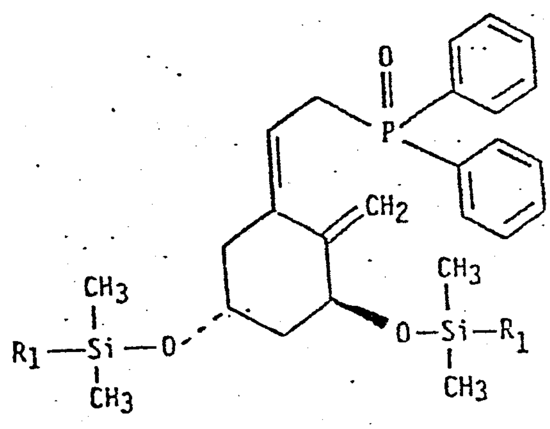

III

worin $R_1$ die obige Bedeutung hat, zu einer Verbindung der Formel

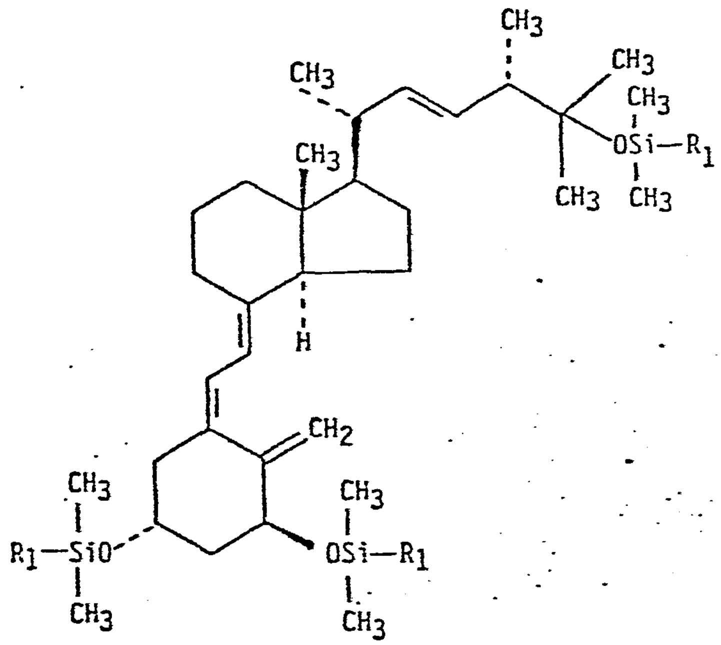

IV

worin $R_1$ die obige Bedeutung hat, umsetzt und

b) die Schutzgruppen aus der Verbindung der Formel IV abspaltet.

2. Praktisch reines kristallines 1α,25-Dihydroxyergocalciferol der Formel

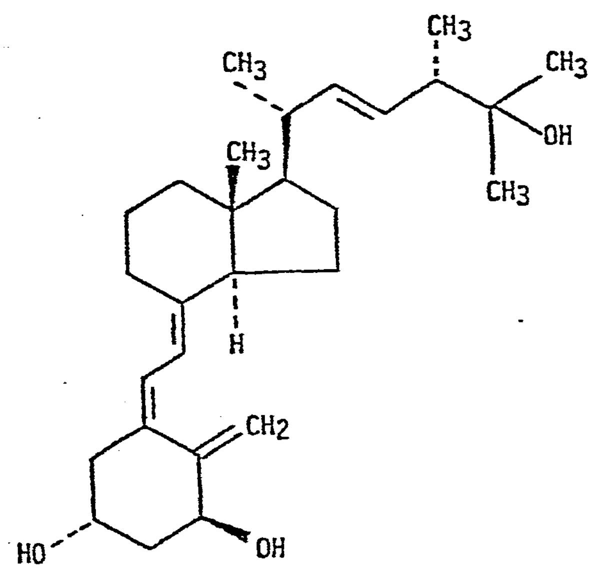

I

3. Die Verbindungen [1R-[1α(R*),3aβ,4α,7aα]]-Octahydro-4-hydroxy-β,7a-dimethyl-1H-inden-1-propannitril und [1R-[1α(S*),3aβ,4α,7aα]]-Octahydro-4-hydroxy-β,7a-dimethyl-1H-inden-1-propanal der Formel

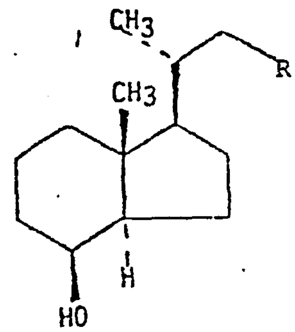

VI

worin R Cyan oder Formyl ist.

4. Eine Verbindung der Formel

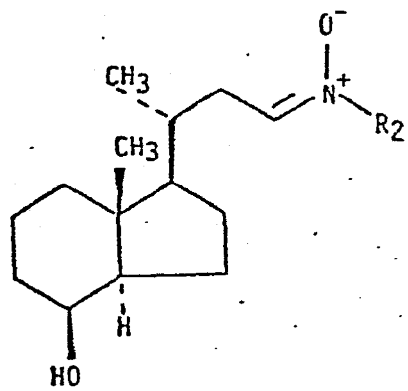

VII

worin $R_2$ nieder-Alkyl, Aryl oder Aryl-nieder-alkyl ist.

5. [1R-[1α(R*,Z),3aβ,4α,7aα]]-Octahydro-7a-methyl-1-[1-methyl-3-(methylimino)propyl]-1H-inden-4-ol-N-oxid.

6. Eine Verbindung der Formel

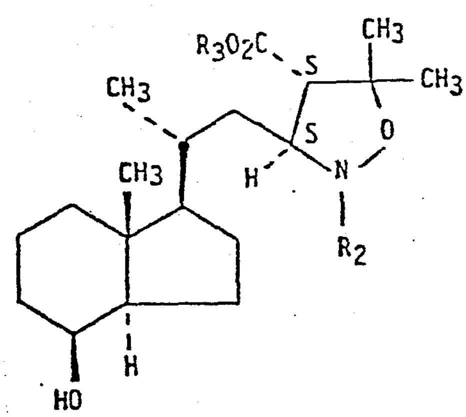

VIII

worin $R_2$ und $R_3$ nieder-Alkyl, Aryl oder Aryl-nieder-alkyl sind.

7. [3S-[3β,4α,3-[(2R*),1R*((1β,3aα,4β,7aβ)]]]-3-[2-(Octahydro-4-hydroxy-7a-methyl-1H-inden-1-yl)propyl]-2,5,5-trimethyl-4-isoxazolidincarbonsäure-methylester.

8. Eine Verbindung der Formel

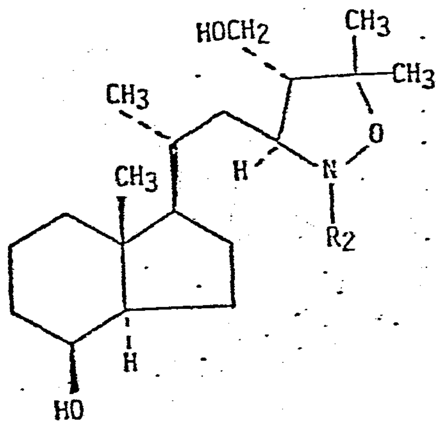

IX

worin $R_2$ nieder-Alkyl, Aryl oder Aryl-nieder-alkyl ist.

9. [3S-[3β,4α,3-[(2R*),1R*(1β,3aα,4β,7aβ)]]]-3-[2-(Octahydro-4-hydroxy-7a-methyl-1H-inden-1-yl)propyl]-2,5,5-trimethyl-4-isoxazolidinmethanol.

10. Eine Verbindung der Formel

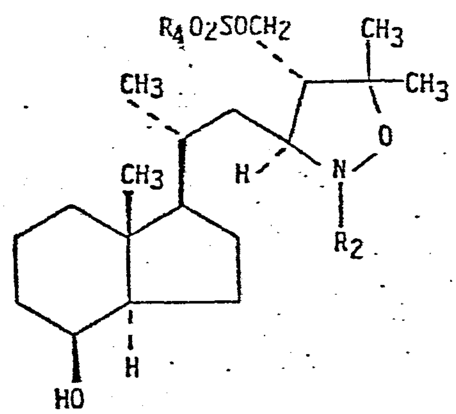

X

worin $R_2$ und $R_4$ nieder-Alkyl, Aryl oder Aryl-nieder-alkyl sind.

11. [1R-[1α(R*),3aβ,4α,7aα,(3S*,4S*)]]-Octahydro-7a-methyl-1-[1-methyl-2-[4-[[[(4-methylphenyl)sulfonyl]oxy]-methyl]-2,5,5-trimethyl-3-isoxazolidinyl]äthyl]-1H-inden-4-ol.

12. Eine Verbindung der Formel

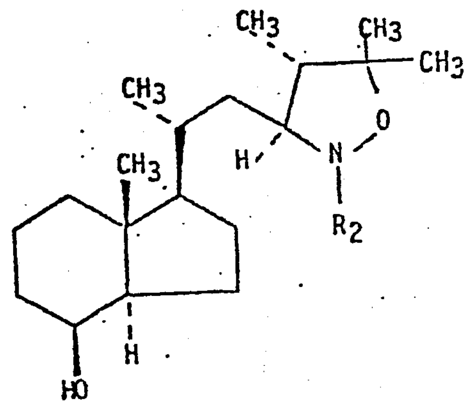

XI

worin $R_2$ nieder-Alkyl, Aryl oder Aryl-nieder-alkyl ist.

13. [1R-[1α(R*),3aβ,4α,7aα,(3S*,4S*)]]-Octahydro-7a-methyl-1-[1-methyl-2-(2,4,5,5-tetramethyl-3-isoxazolidinyl)-äthyl]-1H-inden-4-ol.

14. Eine Verbindung der Formel

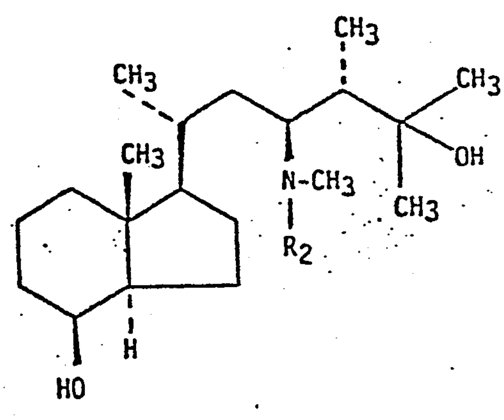

XII

worin $R_2$ nieder-Alkyl, Aryl oder Aryl-nieder-alkyl ist.

15. [1R-[1α(R*,S*,S*),3aβ,4α,7aα]]-Octahydro-γ-(dimethylamino)-4-hydroxy-α,α,β,ε,7a-pentamethyl-1H-inden-1-pentanol.

16. [1R-[(1α(R*),2E,4S*,3aβ,4α,7aα]]-Octahydro-1-(5-hydroxy-1,4,5-trimethyl-2-hexenyl)-7a-methyl-1H-inden-4-ol.

17. [1R-[1α(1R*,2E,4S*),3aβ,7aα]]-Octahydro-1-(5-hydroxy-1,4,5-trimethyl-2-hexenyl)-7a-methyl-4H-inden-4-on.

18. Eine Verbindung der Formel

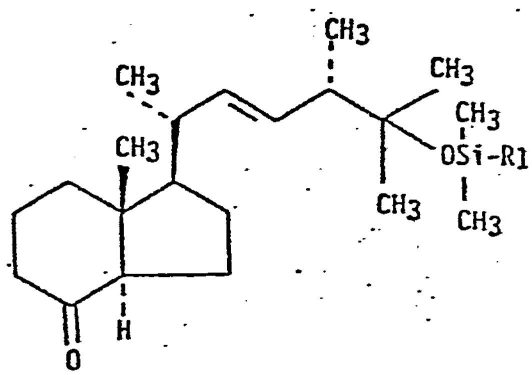

II

worin $R_1$ nieder-Alkyl, Aryl oder Aryl-nieder-alkyl ist.

19. [1R-[1α(1R*,2E,4S*),3aβ,7aα]]-Octahydro-1-(1,4,5-trimethyl-5-trimethylsilyloxy-2-hexenyl)-7a-methyl-4H-inden-4-on.

20. 1α,25-Dihydroxyergocalciferol als Mittel zur Behandlung von Zuständen, die durch zu tiefe 1α,25-Dihydroxycholecalciferol-Spiegel charakterisiert sind, insbesondere von Osteodystrophie und Osteoporose.

21. Arzneimittel auf der Basis von 1α,25-Dihydroxyergocalciferol.

***